# EUROPEAN PATENT SPECIFICATION

(11) **EP 4 051 659 B1**
(45) Date of publication and mention of the grant of the patent: **28.01.2026**
(21) Application number: 20811170.8
(22) Date of filing: 27.10.2020
(51) Int. Cl.: C07C 57/30

(54) **SOLID STATE FORMS OF FEZAGEPRAS AND PROCESS FOR PREPARATION THEREOF**
FESTE FORMEN VON FEZAGEPRAS UND VERFAHREN ZU DEREN HERSTELLUNG
FORMES À L'ÉTAT SOLIDE DE FEZAGEPRAS ET LEUR PROCÉDÉ DE PRÉPARATION

(30) Priority: 31.10.2019 IN 201911044110; 31.10.2019 IN 201911044184
(43) Date of publication of application: 07.09.2022
(73) Proprietor: Assia Chemical Industries Ltd., Tel Aviv 6944020 (IL)
(72) Inventor: MUTHUSAMY, Anantha Rajmohan, Tamil Nadu 626189 (IN); SINGH, Amit, Noida, Uttar Pradesh 201310 (IN); YAZALI, Venkata Subbarao, Guntur, Andhra Pradesh 522309 (IN)
(74) Representative: D Young & Co LLP
(86) International application number: PCT/US2020/057447
(87) International publication number: WO 2021/086805

(56) References cited:
- WO-A1-2010/127440
- WO-A1-2010/127448

## Description

### FIELD OF THE DISCLOSURE

The present disclosure encompasses a crystalline polymorph of Setogepram sodium, processes for preparation thereof, and pharmaceutical compositions thereof.

### BACKGROUND OF THE DISCLOSURE

Setogepram, 2-(3-pentylphenyl)acetic acid, is also known as Fezagepras or PBI 4050), and has the following chemical structure:

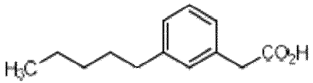

Setogepram is an orally available small molecule, and it is developed for the treatment of Alstrom Syndrome and Idiopathic pulmonary fibrosis. Setogepram has antiinflammatory and anti-fibrotic activity, e.g. due to its ability to reduce fibrosis through regulation of macrophages and fibroblasts, and accordingly may also be useful for the treatment of a number of inflammatory diseases, such as cystic fibrosis, renal fibrosis, liver fibrosis, pancreatic fibrosis, lung fibrosis, liver fibrosis, pulmonary fibrosis, diabetic nephropathy, metabolic syndrome, scleroderma, type 2 diabetes mellitus, acute lung injury, non-alcoholic fatty liver disease, chronic kidney disease, diabetic kidney disease, Crohn's disease, scleroderma, osteoporosis, or pulmonary hypertension.

The compound is described in US Patent No. 8,927,765.

Polymorphism, the occurrence of different crystalline forms, is a property of some molecules and molecular complexes. A single molecule may give rise to a variety of polymorphs having distinct crystal structures and physical properties like melting point, thermal behaviors (e.g., measured by thermogravimetric analysis ("TGA"), or differential scanning calorimetry ("DSC")), X-ray diffraction (XRD) pattern, infrared absorption fingerprint, and solid state (¹³C) NMR spectrum. One or more of these techniques may be used to distinguish different polymorphic forms of a compound.

Different salts and solid state forms (including solvated forms) of an active pharmaceutical ingredient may possess different properties. Such variations in the properties of different salts and solid state forms and solvates may provide a basis for improving formulation, for example, by facilitating better processing or handling characteristics, changing the dissolution profile in a favorable direction, or improving stability (polymorph as well as chemical stability) and shelf-life. These variations in the properties of different salts and solid state forms may also offer improvements to the final dosage form, for instance, if they serve to improve bioavailability. Different salts and solid state forms and solvates of an active pharmaceutical ingredient may also give rise to a variety of polymorphs or crystalline forms, which may in turn provide additional opportunities to assess variations in the properties and characteristics of a solid active pharmaceutical ingredient.

Discovering new solid state forms and solvates of a pharmaceutical product may yield materials having desirable processing properties, such as ease of handling, ease of processing, storage stability, and ease of purification or as desirable intermediate crystal forms that facilitate conversion to other polymorphic forms. New solid state forms of a pharmaceutically useful compound can also provide an opportunity to improve the performance characteristics of a pharmaceutical product. It enlarges the repertoire of materials that a formulation scientist has available for formulation optimization, for example by providing a product with different properties, including a different crystal habit, higher crystallinity, or polymorphic stability, which may offer better processing or handling characteristics, improved dissolution profile, or improved shelf-life (chemical/physical stability). For at least these reasons, there is a need for additional solid state forms (including solvated forms) of Setogepram.

### SUMMARY OF THE DISCLOSURE

The present disclosure relates to crystalline polymorphs or salts of Setogepram, particularly Setogepram sodium, processes for preparation thereof, and pharmaceutical compositions thereof. These crystalline polymorphs can be used to prepare other solid state forms of Setogepram, Setogepram salts, particularly Setogepram sodium and their solid state forms.

The invention is defined in claims 1-15.

The present disclosure also provides uses of the said solid state forms of Setogepram and salts thereof, particularly Setogepram sodium of the present disclosure, in the preparation of other solid state forms of Setogepram and/or Setogepram co-crystals and/or salts, particularly Setogepram sodium thereof, and their solid state forms thereof.

The present disclosure provides crystalline polymorphs of Setogepram and salts of Setogepram, particularly Setogepram sodium for use in medicine, including for the treatment of Alstrom Syndrome and Idiopathic pulmonary fibrosis.

The present disclosure also encompasses the use of crystalline polymorphs of Setogepram and salts of Setogepram, particularly Setogepram sodium of the present disclosure for the preparation of pharmaceutical compositions and/or formulations.

In another aspect, the present disclosure provides pharmaceutical compositions comprising crystalline polymorphs of Setogepram and salts of Setogepram, particularly Setogepram sodium according to the present disclosure.

The present disclosure includes processes for preparing the above mentioned pharmaceutical compositions. The processes include combining any one or a combination of the crystalline polymorphs of Setogepram and salts of Setogepram, particularly Setogepram sodium with at least one pharmaceutically acceptable excipient.

The crystalline polymorph of Setogepram and salts of Setogepram, particularly Setogepram sodium as defined herein and the pharmaceutical compositions or formulations of the crystalline polymorph of Setogepram and salts of Setogepram, particularly Setogepram sodium may be used as medicaments, such as for the treatment of Alstrom Syndrome and Idiopathic pulmonary fibrosis.

The present disclosure also provides methods of treating Alstrom Syndrome and Idiopathic pulmonary fibrosis, by administering a therapeutically effective amount of any one or a combination of the crystalline polymorphs of Setogepram and salts of Setogepram, particularly Setogepram sodium of the present disclosure, or at least one of the above pharmaceutical compositions, to a subject suffering from Alstrom Syndrome and Idiopathic pulmonary fibrosis, or otherwise in need of the treatment.

The present disclosure also provides uses of crystalline polymorphs of Setogepram and salts of Setogepram, particularly Setogepram sodium of the present disclosure, or at least one of the above pharmaceutical compositions, for the manufacture of medicaments for treating e.g. Alstrom Syndrome and Idiopathic pulmonary fibrosis.

### BRIEF DESCRIPTION OF THE DRAWINGS

Figure 1 shows a characteristic X-ray powder diffraction pattern (XRPD) of Setogepram sodium Form ST2.
Figure 2 shows a characteristic X-ray powder diffraction pattern (XRPD) of Setogepram sodium Form ST3.
Figure 3a shows ¹³C solid state NMR spectrum of Form ST2 of Setogepram sodium (full scan).
Figure 3b shows ¹³C solid state NMR spectrum of Form ST2 of Setogepram sodium (at the range of 0-100 ppm).
Figure 3c shows ¹³C solid state NMR spectrum of Form ST2 of Setogepram sodium (at the range of 100-200 ppm).

### DETAILED DESCRIPTION OF THE DISCLOSURE

The name of the active agent, Setogepram, has been changed to Fezagepras. As used herein, references to Setogepram are understood to be synonymous with Fezagepras, each of which refers to the compound 2-(3-pentylphenyl)acetic acid.

The present disclosure encompasses solid state forms of Setogepram, including crystalline polymorphs of Setogepram and salts of Setogepram, particularly Setogepram sodium, processes for preparation thereof, and pharmaceutical compositions thereof.

In embodiments, the present disclosure provides a crystalline form of Setogepram sodium designated as Form ST2 (defined herein).

Solid state properties of Setogepram and salts of Setogepram, particularly Setogepram sodium and crystalline polymorphs thereof can be influenced by controlling the conditions under which Setogepram and salts of Setogepram, particularly Setogepram sodium and crystalline polymorphs thereof are obtained in solid form.

A solid state form (or polymorph) may be referred to herein as polymorphically pure or as substantially free of any other solid state (or polymorphic) forms. As used herein in this context, the expression "substantially free of any other forms" will be understood to mean that the solid state form contains about 20% (w/w) or less, about 10% (w/w) or less, about 5% (w/w) or less, about 2% (w/w) or less, about 1% (w/w) or less, or about 0% of any other forms of the subject compound as measured, for example, by XRPD. Thus, a crystalline polymorph of Setogepram and salts of Setogepram, particularly Setogepram sodium described herein as substantially free of any other solid state forms would be understood to contain greater than about 80% (w/w), greater than about 90% (w/w), greater than about 95% (w/w), greater than about 98% (w/w), greater than about 99% (w/w), or about 100% of the subject crystalline polymorph of Setogepram. In some embodiments of the disclosure, the described crystalline polymorph of Setogepram may contain from about 1% to about 20% (w/w), from about 5% to about 20% (w/w), or from about 5% to about 10% (w/w) of one or more other crystalline polymorph of the same Setogepram.

Depending on which other crystalline polymorphs a comparison is made, the crystalline polymorphs of Setogepram of the present disclosure may have advantageous properties selected from at least one of the following: chemical purity, flowability, solubility, dissolution rate, morphology or crystal habit, stability, such as chemical stability as well as thermal and mechanical stability with respect to polymorphic conversion, stability towards dehydration and/or storage stability, low content of residual solvent, a lower degree of hygroscopicity, flowability, and advantageous processing and handling characteristics such as compressibility and bulk density.

A solid state form, such as a crystal form or an amorphous form, may be referred to herein as being characterized by graphical data "as depicted in" or "as substantially depicted in" a Figure. Such data include, for example, powder X-ray diffractograms and solid state NMR spectra. As is well-known in the art, the graphical data potentially provides additional technical information to further define the respective solid state form (a so-called "fingerprint") which cannot necessarily be described by reference to numerical values or peak positions alone. In any event, the skilled person will understand that such graphical representations of data may be subject to small variations, e.g., in peak relative intensities and peak positions due to certain factors such as, but not limited to, variations in instrument response and variations in sample concentration and purity, which are well known to the skilled person. Nonetheless, the skilled person would readily be capable of comparing the graphical data in the Figures herein with graphical data generated for an unknown crystal form and confirm whether the two sets of graphical data are characterizing the same crystal form or two different crystal forms. A crystal form of Setogepram and salts of Setogepram, particularly Setogepram sodium referred to herein as being characterized by graphical data "as depicted in" or "as substantially depicted in" a Figure will thus be understood to include any crystal forms of Setogepram and salts of Setogepram, particularly Setogepram sodium characterized with the graphical data having such small variations, as are well known to the skilled person, in comparison with the Figure.

As used herein, and unless stated otherwise, the term "anhydrous" in relation to crystalline forms of Setogepram, relates to a crystalline form of Setogepram and salts of Setogepram, particularly Setogepram sodium which does not include any crystalline water (or other solvents) in a defined, stoichiometric amount within the crystal. Moreover, an "anhydrous" form would generally not contain more than 1% (w/w), of either water or organic solvents as measured for example by TGA.

The term "solvate," as used herein and unless indicated otherwise, refers to a crystal form that incorporates a solvent in the crystal structure. When the solvent is water, the solvate is often referred to as a "hydrate." The solvent in a solvate may be present in either a stoichiometric or in a non-stoichiometric amount.

As used herein, the term "isolated" in reference to crystalline polymorph of Setogepram and salts of Setogepram, particularly Setogepram sodium of the present disclosure corresponds to a crystalline polymorph of Setogepram and salts of Setogepram, particularly Setogepram sodium that is physically separated from the reaction mixture in which it is formed.

As used herein, unless stated otherwise, the XRPD measurements are taken using copper Kα radiation wavelength 1.54187 Å. XRPD peaks reported herein are measured using CuK α radiation, λ = 1.54187 Å, typically at a temperature of 25 ± 3°C.

A thing, *e.g.,* a reaction mixture, may be characterized herein as being at, or allowed to come to "room temperature" or "ambient temperature", often abbreviated as "RT." This means that the temperature of the thing is close to, or the same as, that of the space, *e.g.,* the room or fume hood, in which the thing is located. Typically, room temperature is from about 20°C to about 30°C, or about 22°C to about 27°C, or about 25°C.

The amount of solvent employed in a chemical process, e.g., a reaction or crystallization, may be referred to herein as a number of "volumes" or "vol" or "V." For example, a material may be referred to as being suspended in 10 volumes (or 10 vol or 10V) of a solvent. In this context, this expression would be understood to mean milliliters of the solvent per gram of the material being suspended, such that suspending a 5 grams of a material in 10 volumes of a solvent means that the solvent is used in an amount of 10 milliliters of the solvent per gram of the material that is being suspended or, in this example, 50 mL of the solvent. In another context, the term "v/v" may be used to indicate the number of volumes of a solvent that are added to a liquid mixture based on the volume of that mixture. For example, adding solvent X (1.5 v/v) to a 100 ml reaction mixture would indicate that 150 mL of solvent X was added.

A process or step may be referred to herein as being carried out "overnight." This refers to a time interval, e.g., for the process or step, that spans the time during the night, when that process or step may not be actively observed. This time interval is from about 8 to about 20 hours, or about 10-18 hours, in some cases about 16 hours.

As used herein, the term "reduced pressure" refers to a pressure that is less than atmospheric pressure. For example, reduced pressure is about 10 mbar to about 50 mbar.

As used herein and unless indicated otherwise, the term "ambient conditions" refer to atmospheric pressure and a temperature of 22-24°C.

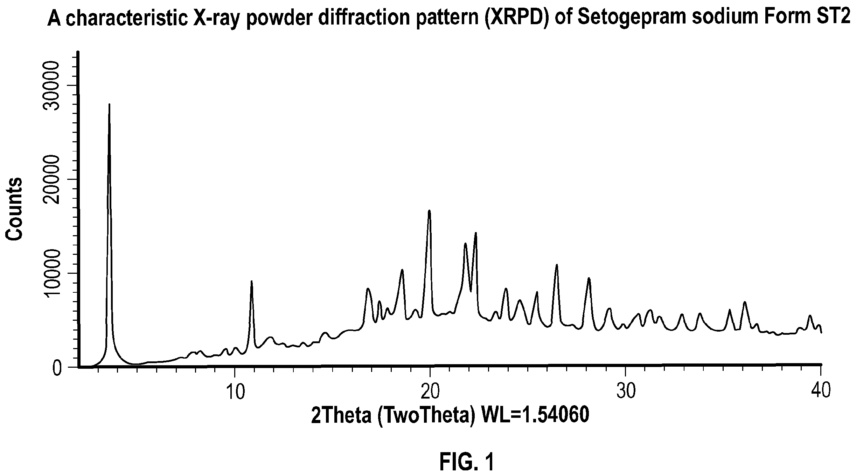

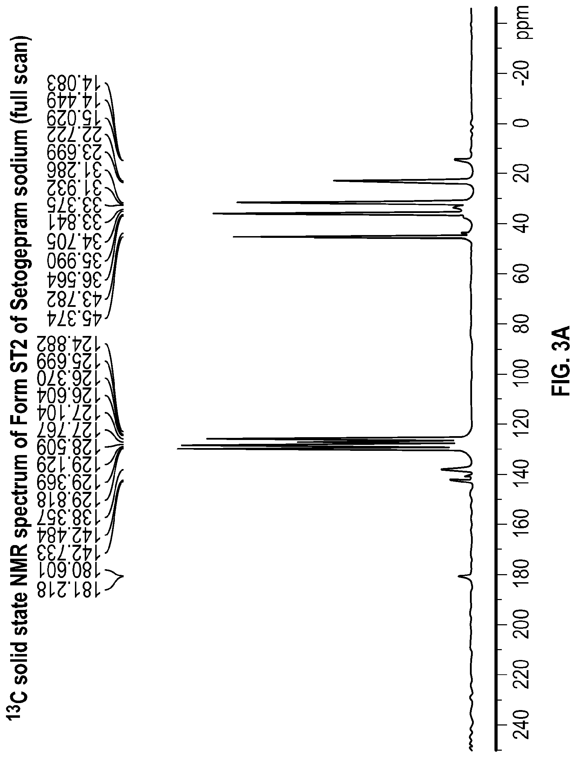

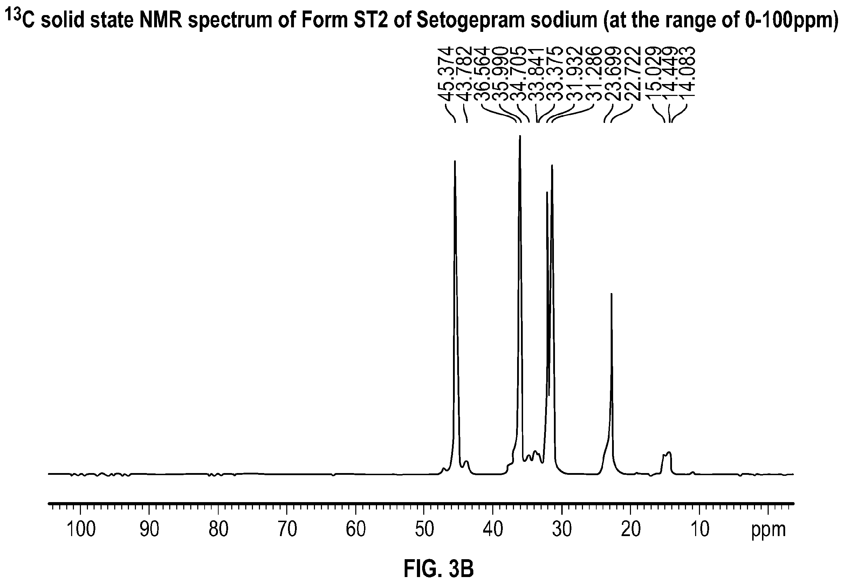

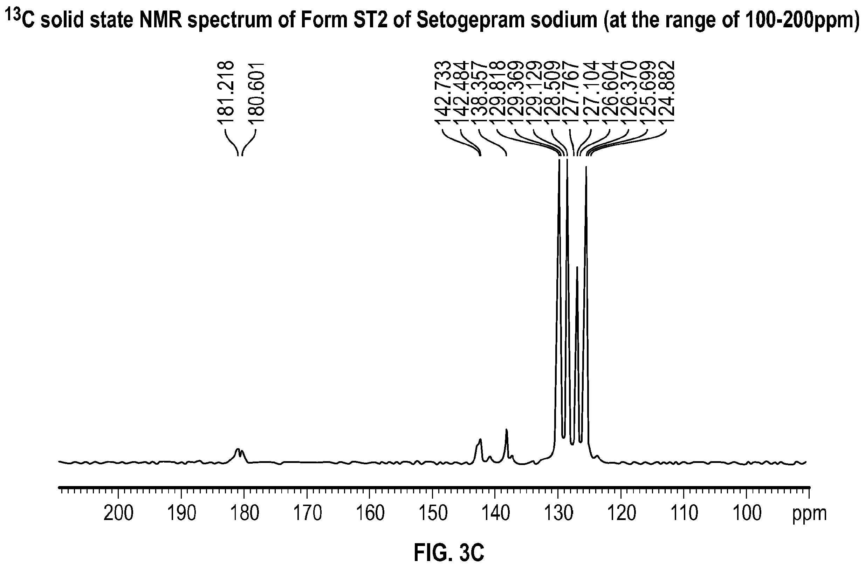

The present invention relates to a crystalline polymorph of Setogepram Sodium, designated ST2, which is characterized by data selected from one or more of the following: an X-ray powder diffraction pattern substantially as depicted in Figure 1; an X-ray powder diffraction pattern having peaks at 3.6, 10.9, 19.9, 26.5 and 28.1 degrees 2-theta ± 0.2 degrees 2-theta;. a solid state ¹³C NMR spectrum having peaks at 22.7, 31.3, 36.0, 125.7, 128.5 and 129.8 ppm ± 0.2 ppm; a solid state ¹³C NMR spectrum having the following chemical shift absolute differences from a reference peak at 45.4 ppm ± 2 ppm of 22.7, 14.1, 9.4, 80.3, 83.1 and 84.4 ppm ± 0.1 ppm; a solid state ¹³C NMR spectrum substantially as depicted in Figures 3a, 3b or 3c; and combinations of these data.

Crystalline Form ST2 of Setogepram sodium may be further characterized by an X-ray powder diffraction pattern having peaks at 3.6, 10.9, 19.9, 26.5 and 28.1 degrees 2-theta ± 0.2 degrees 2-theta, and also having any one, two, three, four or five additional peaks selected from 18.6, 21.8, 25.5, 28.1 and 29.2 degrees 2-theta ± 0.2 degrees 2-theta.

Crystalline Form ST2 of Setogepram sodium may be alternatively characterized by an X-ray powder diffraction pattern having peaks at 3.6, 10.9, 18.6, 19.9, 21.8, 25.5, 26.5, 28.1 and 29.2 degrees 2-theta ± 0.2 degrees 2-theta.

Alternatively, crystalline Form ST2 of Setogepram Sodium is characterized by data selected from one or more of the following: an X-ray powder diffraction pattern substantially as depicted in Figure 1; an X-ray powder diffraction pattern having peaks at 3.6, 10.9, 19.9, and 26.5 degrees 2-theta ± 0.2 degrees 2-theta; a solid state ¹³C NMR spectrum having peaks at 22.7, 31.3, 36.0, 125.7, 128.5 and 129.8 ppm ± 0.2 ppm; a solid state ¹³C NMR spectrum having the following chemical shift absolute differences from a reference peak at 45.4 ppm ± 2 ppm of 22.7, 14.1, 9.4, 80.3, 83.1 and 84.4 ppm ± 0.1 ppm; a solid state ¹³C NMR spectrum substantially as depicted in Figures 3a, 3b or 3c; and combinations of these data. Crystalline Form ST2 of Setogepram sodium may be further characterized by an X-ray powder diffraction pattern having peaks at 3.6, 10.9, 19.9, and 26.5 degrees 2-theta ± 0.2 degrees 2-theta, and also having any one, two, three, four or five additional peaks selected from 18.6, 21.8, 25.5, 28.1 and 29.2 degrees 2-theta ± 0.2 degrees 2-theta.

In one embodiment of the present disclosure, crystalline Form ST2 of Setogepram sodium is isolated.

Crystalline Form ST2 of Setogepram sodium may be hydrate form. More preferably, Crystalline Form ST2 of Setogepram sodium is sesquihydate form. Typically, the water content in crystalline Form ST2 of Setogepram sodium is from about 8.0 % to about 11 % (w/w) measured by typical method such as TGA.

Crystalline Form ST2 of Setogepram sodium may be characterized by each of the above characteristics alone/or by all possible combinations, e.g., an XRPD pattern having peaks at 3.6, 10.9, 19.9, 26.5 and 28.1 degrees 2-theta ± 0.2 degrees 2-theta; an XRPD pattern as depicted in Figure 1, and combinations thereof.

The present invention further relates to a process for preparation of Form ST2 of Setogepram sodium. The process comprises:
(i) providing a solution of Setogepram sodium in methanol;
(ii) removing the methanol;
(iii) adding methyl tert-butyl ether, and removing the solvent to obtain a solid;
(iv) cooling the solid; and
(v) optionally drying.

In the process, the solution in step (i) may be at a temperature of about 18°C to about 40°C, or about 20°C to about 35°C, or about 25°C to about 30°C .

In the process, step (ii) may comprise distilling the solution at a temperature of about 50°C to about 55°C, or about 40°C to about 45°C, preferably wherein the distilling is conducted under reduced pressure. The distillation may be conducted for a sufficient time to remove most of the solvent, and optionally to form a solid. Particularly, the distillation may be carried out over a period of about 30 minutes to about 2 hours, about 45 minutes to about 1.5 hours, or about 1 hour.

In this process, methanol in step (i) is typically used in an amount of about 1 ml to about 4 ml, about 1.5 ml to about 3.5 ml, or about 2 ml, per gram of Setogepram sodium. In any embodiment of the process, methyl tert-butyl ether in step (iii) may be used in an amount of about 8 ml to about 12 ml, about 9 ml to about 11 ml, or about 10 ml, per gram of Setogepram sodium.

In this process, step (iii) may comprise distilling the solvent for a sufficient time to remove most of the solvent, and optionally to form a solid. Particularly, the distillation may be carried out over a period of about 25 minutes to 40 minutes, or 30 minutes to 35 minutes.

In this process, step (iv) may comprise cooling the resulting solid, typically to a temperature of about 5°C to about -15°C , about 3°C to about -12°C , or about 0°C to about - 10°C. The solid may be cooled over a period time to form crystalline Setogepram sodium Form ST2. Particularly, the cooling may be conducted over a period of about 12 hours to about 18 hours, about 14 hours to about 17 hours, or about 16 hours.

In this process, the crystalline Form ST2 of Setogepram sodium may be dried. The crystalline Form ST2 of Setogepram sodium may be dried under vacuum, typically at a temperature of about 20°C to about 60°C, about 25°C to about 55°C, about 25°C to about 40°C, or about 30°C. The drying may be carried out for any suitable time to remove the solvent, typically about 1 to about 5 hours, about 1.5 hours to about 4 hours, or about 2 hours to about 3 hours.

In the process, there is provided a further step of combining the crystalline form ST2 with at least one pharmaceutically acceptable excipient to prepare a pharmaceutical composition or dosage form.

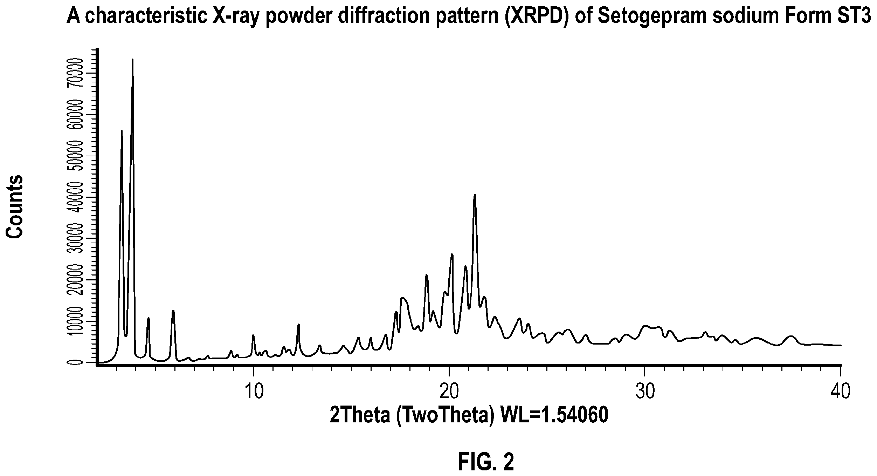

A crystalline polymorph of Setogepram Sodium, designated ST3 is also disclosed. The crystalline Form ST3 of Setogepram Sodium may be characterized by data selected from one or more of the following: an X-ray powder diffraction pattern substantially as depicted in Figure 2; an X-ray powder diffraction pattern having peaks at 3.8, 4.6, 5.9, 12.3 and 21.3 degrees 2-theta ± 0.2 degrees 2-theta; and combinations of these data.

Crystalline Form ST3 of Setogepram sodium may be further characterized by an X-ray powder diffraction pattern having peaks at 3.8, 4.6, 5.9, 12.3 and 21.3 degrees 2-theta ± 0.2 degrees 2-theta, and also having any one, two, three, four or five additional peaks selected from 16.0, 16.7, 17.2, 18.8 and 20.8 degrees 2-theta ± 0.2 degrees 2-theta.

Crystalline Form ST3 of Setogepram sodium may be further characterized by an X-ray powder diffraction pattern having peaks at 3.8, 4.6, 5.9, 12.3, 16.0, 16.7, 17.2, 18.8, 20.8 and 21.3 degrees 2-theta ± 0.2 degrees 2-theta.

Crystalline Form ST3 of Setogepram sodium is isolated.

Crystalline Form ST3 of Setogepram sodium may be characterized by each of the above characteristics alone/or by all possible combinations, e.g., an XRPD pattern having peaks at 3.8, 4.6, 5.9, 12.3 and 21.3 degrees 2-theta ± 0.2 degrees 2-theta; an XRPD pattern as depicted in Figure 2, and combinations thereof.

The above crystalline polymorphs can be used to prepare other crystalline polymorphs of Setogepram, Setogepram salts, particularly Setogepram sodium and their solid state forms.

The present disclosure encompasses a process for preparing other solid state forms of Setogepram, Setogepram salts, particularly Setogepram sodium and their solid state forms thereof. The process includes preparing any one of the Setogepram salts, particularly Setogepram sodium and solid state forms of Setogepram by the processes of the present disclosure, and converting that salt to said other Setogepram salt, particularly Setogepram sodium.

The present disclosure provides the above described crystalline polymorphs of Setogepram for use in the preparation of pharmaceutical compositions comprising Setogepram and/or crystalline polymorphs thereof.

The present disclosure also encompasses the use of crystalline polymorphs of Setogepram of the present disclosure for the preparation of pharmaceutical compositions of crystalline polymorph Setogepram and/or crystalline polymorphs thereof.

The present disclosure includes processes for preparing the above mentioned pharmaceutical compositions. The processes include combining any one or a combination of the crystalline polymorphs of Setogepram of the present disclosure with at least one pharmaceutically acceptable excipient.

Pharmaceutical combinations or formulations of the present disclosure contain any one or a combination of the solid state forms of Setogepram of the present disclosure. In addition to the active ingredient, the pharmaceutical formulations of the present disclosure can contain one or more excipients. Excipients are added to the formulation for a variety of purposes.

Diluents increase the bulk of a solid pharmaceutical composition, and can make a pharmaceutical dosage form containing the composition easier for the patient and caregiver to handle. Diluents for solid compositions include, for example, microcrystalline cellulose (e.g. Avicel^{®}), microfine cellulose, lactose, starch, pregelatinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, polymethacrylates (e.g. Eudragit^{®}), potassium chloride, powdered cellulose, sodium chloride, sorbitol, and talc.

Solid pharmaceutical compositions that are compacted into a dosage form, such as a tablet, can include excipients whose functions include helping to bind the active ingredient and other excipients together after compression. Binders for solid pharmaceutical compositions include acacia, alginic acid, carbomer (e.g. carbopol), carboxymethylcellulose sodium, dextrin, ethyl cellulose, gelatin, guar gum, hydrogenated vegetable oil, hydroxyethyl cellulose, hydroxypropyl cellulose (e.g. Klucel^{®}), hydroxypropyl methyl cellulose (e.g. Methocel^{®}), liquid glucose, magnesium aluminum silicate, maltodextrin, methylcellulose, polymethacrylates, povidone (e.g. Kollidon^{®}, Plasdone^{®}), pregelatinized starch, sodium alginate, and starch.

The dissolution rate of a compacted solid pharmaceutical composition in the patient's stomach can be increased by the addition of a disintegrant to the composition. Disintegrants include alginic acid, carboxymethylcellulose calcium, carboxymethylcellulose sodium (e.g. Ac-Di-Sol^{®}, Primellose^{®}), colloidal silicon dioxide, croscarmellose sodium, crospovidone (e.g. Kollidon^{®}, Polyplasdone^{®}), guar gum, magnesium aluminum silicate, methyl cellulose, microcrystalline cellulose, polacrilin potassium, powdered cellulose, pregelatinized starch, sodium alginate, sodium starch glycolate (e.g. Explotab^{®}), and starch.

Glidants can be added to improve the flowability of a non-compacted solid composition and to improve the accuracy of dosing. Excipients that can function as glidants include colloidal silicon dioxide, magnesium trisilicate, powdered cellulose, starch, talc, and tribasic calcium phosphate.

When a dosage form such as a tablet is made by the compaction of a powdered composition, the composition is subjected to pressure from a punch and dye. Some excipients and active ingredients have a tendency to adhere to the surfaces of the punch and dye, which can cause the product to have pitting and other surface irregularities. A lubricant can be added to the composition to reduce adhesion and ease the release of the product from the dye. Lubricants include magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl fumarate, stearic acid, talc, and zinc stearate.

Flavoring agents and flavor enhancers make the dosage form more palatable to the patient. Common flavoring agents and flavor enhancers for pharmaceutical products that can be included in the composition of the present disclosure include maltol, vanillin, ethyl vanillin, menthol, citric acid, fumaric acid, ethyl maltol, and tartaric acid.

Solid and liquid compositions can also be dyed using any pharmaceutically acceptable colorant to improve their appearance and/or facilitate patient identification of the product and unit dosage level.

In liquid pharmaceutical compositions of the present invention, Setogepram and any other solid excipients can be dissolved or suspended in a liquid carrier such as water, vegetable oil, alcohol, polyethylene glycol, propylene glycol, or glycerin.

Liquid pharmaceutical compositions can contain emulsifying agents to disperse uniformly throughout the composition an active ingredient or other excipient that is not soluble in the liquid carrier. Emulsifying agents that can be useful in liquid compositions of the present invention include, for example, gelatin, egg yolk, casein, cholesterol, acacia, tragacanth, chondrus, pectin, methyl cellulose, carbomer, cetostearyl alcohol, and cetyl alcohol.

Liquid pharmaceutical compositions of the present invention can also contain a viscosity enhancing agent to improve the mouth-feel of the product and/or coat the lining of the gastrointestinal tract. Such agents include acacia, alginic acid bentonite, carbomer, carboxymethylcellulose calcium or sodium, cetostearyl alcohol, methyl cellulose, ethylcellulose, gelatin guar gum, hydroxyethyl cellulose, hydroxypropyl cellulose, hydroxypropyl methyl cellulose, maltodextrin, polyvinyl alcohol, povidone, propylene carbonate, propylene glycol alginate, sodium alginate, sodium starch glycolate, starch tragacanth, xanthan gum and combinations thereof.

Sweetening agents such as sorbitol, saccharin, sodium saccharin, sucrose, aspartame, fructose, mannitol, and invert sugar can be added to improve the taste.

Preservatives and chelating agents such as alcohol, sodium benzoate, butylated hydroxyl toluene, butylated hydroxyanisole, and ethylenediamine tetraacetic acid can be added at levels safe for ingestion to improve storage stability.

According to the present disclosure, a liquid composition can also contain a buffer such as gluconic acid, lactic acid, citric acid, or acetic acid, sodium gluconate, sodium lactate, sodium citrate, or sodium acetate. Selection of excipients and the amounts used can be readily determined by the formulation scientist based upon experience and consideration of standard procedures and reference works in the field.

The solid compositions of the present disclosure include powders, granulates, aggregates, and compacted compositions. The dosages include dosages suitable for oral, buccal, rectal, parenteral (including subcutaneous, intramuscular, and intravenous), inhalant, and ophthalmic administration. Although the most suitable administration in any given case will depend on the nature and severity of the condition being treated, the route of administration may be oral. The dosages can be conveniently presented in unit dosage form and prepared by any of the methods well-known in the pharmaceutical arts.

Dosage forms include solid dosage forms like tablets, powders, capsules, suppositories, sachets, troches, and lozenges, as well as liquid syrups, suspensions, and elixirs.

The dosage form of the present disclosure can be a capsule containing the composition, such as a powdered or granulated solid composition of the disclosure, within either a hard or soft shell. The shell can be made from gelatin and optionally contain a plasticizer such as glycerin and/or sorbitol, an opacifying agent and/or colorant.

The active ingredient and excipients can be formulated into compositions and dosage forms according to methods known in the art.

A composition for tableting or capsule filling can be prepared by wet granulation. **In** wet granulation, some or all of the active ingredients and excipients in powder form are blended and then further mixed in the presence of a liquid, typically water, that causes the powders to clump into granules. The granulate is screened and/or milled, dried, and then screened and/or milled to the desired particle size. The granulate can then be tableted, or other excipients can be added prior to tableting, such as a glidant and/or a lubricant.

A tableting composition can be prepared conventionally by dry blending. For example, the blended composition of the actives and excipients can be compacted into a slug or a sheet and then comminuted into compacted granules. The compacted granules can subsequently be compressed into a tablet.

As an alternative to dry granulation, a blended composition can be compressed directly into a compacted dosage form using direct compression techniques. Direct compression produces a more uniform tablet without granules. Excipients that are particularly well suited for direct compression tableting include microcrystalline cellulose, spray dried lactose, dicalcium phosphate dihydrate, and colloidal silica. The proper use of these and other excipients in direct compression tableting is known to those in the art with experience and skill in particular formulation challenges of direct compression tableting.

A capsule filling of the present disclosure can include any of the aforementioned blends and granulates that were described with reference to tableting, but they are not subjected to a final tableting step.

A pharmaceutical formulation of Setogepram can be administered. Setogepram may be formulated for administration to a mammal, particularly to a human, by injection. Setogepram can be formulated, for example, as a viscous liquid solution or suspension, such as a clear solution, for injection. The formulation can contain one or more solvents. A suitable solvent can be selected by considering the solvent's physical and chemical stability at various pH levels, viscosity (which would allow for syringeability), fluidity, boiling point, miscibility, and purity. Suitable solvents include alcohol USP, benzyl alcohol NF, benzyl benzoate USP, and Castor oil USP. Additional substances can be added to the formulation such as buffers, solubilizers, and antioxidants, among others. Ansel et al., Pharmaceutical Dosage Forms and Drug Delivery Systems, 7th ed.

The crystalline polymorphs of Setogepram and the pharmaceutical compositions and/or formulations of Setogepram of the present disclosure can be used as medicaments, particularly in the treatment of Alstrom Syndrome and Idiopathic pulmonary fibrosis.

The present disclosure also provides methods of treating Alstrom Syndrome and Idiopathic pulmonary fibrosis by administering a therapeutically effective amount of any one or a combination of the crystalline polymorphs of Setogepram of the present disclosure, or at least one of the above pharmaceutical compositions and/or formulations, to a subject in need of the treatment.

The Examples are set forth to aid in understanding the disclosure but are not intended to, and should not be construed to limit its scope in any way.

### Powder X-ray Diffraction ("XRPD") method

X-ray diffraction was performed on X-Ray powder diffractometer:
Bruker D8 Advance; Copper Kα radiation (λ = 1.5418 Å); Lynx eye detector; laboratory temperature 22-25 °C; PMMA specimen holder ring. Prior to analysis, the samples were gently ground by means of mortar and pestle in order to obtain a fine powder. The ground sample was adjusted into a cavity of the sample holder and the surface of the sample was smoothed by means of a cover glass.

### Measurement parameters:

Scan range: 2 - 40 degrees 2-theta;
Scan mode: continuous;
Step size: 0.05 degrees;
Time per step: 0.5 s;
Sample spin: 30 rpm;
Sample holder: PMMA specimen holder ring.
All X-Ray Powder Diffraction peak values are calibrated with regard to standard silicon spiking in the sample.

### Procedure for Thermo Gravimetric Analysis (TGA)

Thermogravimetric analysis was conducted on a TA instrument Q500 thermogravimetric analyzer. About 8.3 mg sample was placed into a tared TGA crucible and placed into a TGA furnace. The furnace was heated under nitrogen at a heating rate of 10°C/min up to 300°C.

### Examples

### Preparation of starting materials

Setogepram Sodium can be prepared according to methods known from the literature, for example U.S. Patent No. 8,927,765.

### Example 1: Preparation of Setogepram Sodium Form ST2

Setogepram Sodium (1.0 grams) was dissolved in methanol (2.0 mL) at temperature of about 25°C to about 30°C in a 50 mL round bottom flask. The clear solution was distilled under reduced pressure at temperature of about 40°C to about 45°C for a period of about an hour and a gummy mass was obtained. Methyl tert-butyl ether (10 mL) was added to obtained gummy mass and distilled again for a period of about 30 minutes to 35 minutes. The obtained solid mass was cooled down to temperature of about 0°C to about -10°C for a period of about 16 hours. The obtained solid mass was dried under vacuum at 30°C for a period of about 2 hours to 3 hours. Crystalline Setogepram sodium was obtained. A sample was analyzed by XRPD. Form ST2 was obtained. An XRPD pattern is shown in Figure 1.

### Example 2: Preparation of Setogepram Sodium Form ST3 (reference example)

Setogepram Sodium (5.0 grams) was dissolved in ethanol (25.0 mL) at temperature of about 25°C to about 30°C in a 100 mL round bottom flask. The solution was filtered to remove any undissolved particulate. The clear solution was distilled under reduced pressure (below 50 mbar) at temperature of about 40°C to about 45°C for a period of about 3 hours to 4 hours. The obtained solid was dried in Air Tray Dryer (ATD) at temperature of about 50°C for a period of about 6 hours to 8 hours. Crystalline Setogepram sodium was obtained. A sample was analyzed by XRPD. Form ST3 was obtained (4.6 grams). An XRPD pattern is shown in Figure 2.

## Claims

1. Crystalline Form ST2 of Setogepram sodium, 2-(3-pentylphenyl)acetic acid sodium salt, which is **characterized by** data selected from one or more of the following:
a. an XRPD pattern having peaks at 3.6, 10.9, 19.9, 26.5 and 28.1 degrees 2-theta ± 0.2 degrees 2-theta or an XRPD pattern having peaks at 3.6, 10.9, 19.9, 26.5 and 28.1 degrees 2-theta ± 0.2 degrees 2-theta;
b. an XRPD pattern having peaks at 3.6, 10.9, 19.9, 26.5 degrees 2-theta ± 0.2 degrees 2-theta or an XRPD pattern having peaks at 3.6, 10.9, 19.9, 26.5 and 28.1 degrees 2-theta ± 0.2 degrees 2-theta.
c. an XRPD pattern as depicted in Figure 1;
d. a solid state ¹³C NMR spectrum having peaks at 22.7, 31.3, 36.0, 125.7, 128.5 and 129.8 ppm ± 0.2 ppm;
e. a solid state ¹³C NMR spectrum having the following chemical shift absolute differences from a reference peak at 45.4 ppm ± 2 ppm of 22.7, 14.1, 9.4, 80.3, 83.1 and 84.4 ppm ± 0.1 ppm;
f. a solid-state ¹³C NMR spectrum substantially as depicted in Figures 3a, 3b or 3c; and
g. combinations of two or more of: a, c, d, e, and f; or combinations of two or more of: b, c, d, e and f.

2. Crystalline Form ST2 of Setogepram sodium according to Claim 1, which is **characterized by** an XRPD pattern having peaks at 3.6, 10.9, 19.9, 26.5 and 28.1 degrees 2-theta ± 0.2 degrees 2-theta, and also having one, two, three, four, or five additional peaks selected from 18.6, 21.8, 25.5, 28.1 and 29.2 degrees two theta ± 0.2 degrees two theta.

3. Crystalline Form ST2 of Setogepram sodium according to Claim 1, **characterized by** an XRPD pattern having peaks at 3.6, 10.9, 19.9 and 26.5 degrees 2-theta ± 0.2 degrees 2-theta, and also having one, two, three, four, or five additional peaks selected from 18.6, 21.8, 25.5, 28.1 and 29.2 degrees two theta ± 0.2 degrees two theta.

4. Crystalline Form ST2 of Setogepram sodium according to any of Claims 1, 2 or 3, which is **characterized by** an XRPD pattern having peaks at: 3.6, 10.9, 18.6, 19.9, 21.8, 25.5, 26.5, 28.1, 29.2 degrees 2-theta ± 0.2 degrees 2-theta.

5. Crystalline Form ST2 of Setogepram sodium according to any of Claims 1 to 4, wherein said crystalline form is a hydrate form, optionally wherein the crystalline form contains about 8.0% to about 11% (w/w) of water, or wherein the crystalline form is a sesquihydrate.

6. Crystalline Setogepram sodium according to any of Claims 1 to 5, which contains: no more than about 20%, no more than about 10%, no more than about 5%, no more than about 2%, no more than about 1% or about 0% of any other crystalline forms of Setogepram sodium.

7. Crystalline Setogepram sodium according to any of Claims 1 to 6, which contains: no more than about 20%, no more than about 10%, no more than about 5%, no more than about 2%, no more than about 1% or about 0% of amorphous Setogepram sodium.

8. A pharmaceutical composition comprising crystalline Form ST2 of Setogepram sodium according to any of Claims 1 to 7.

9. Use of crystalline Form ST2 of Setogepram sodium according to any of Claims 1 to 7 for the preparation of a pharmaceutical composition and/or pharmaceutical formulation, preferably wherein the pharmaceutical formulation is oral formulation.

10. A pharmaceutical formulation comprising crystalline Form ST2 of Setogepram sodium according to any of Claims 1 to 7, or a pharmaceutical composition of Claim 8, with at least one pharmaceutically acceptable excipient.

11. A process for preparing a pharmaceutical formulation according to Claim 10, comprising combining crystalline Form ST2 of Setogepram sodium according to any of Claims 1 to 7 or a pharmaceutical composition of Claim 8, with at least one pharmaceutically acceptable excipient.

12. Crystalline Form ST2 of Setogepram sodium, according to any one of Claims 1 to 7, a pharmaceutical composition according to Claim 8, or a pharmaceutical formulation according to Claim 10, for use as a medicament.

13. Crystalline Form ST2 of Setogepram sodium, according to any one of Claims 1 to 7, a pharmaceutical composition according to Claim 8, or a pharmaceutical formulation according to Claim 10, for use in the treatment of: Alstrom Syndrome, Idiopathic pulmonary fibrosis, cystic fibrosis, renal fibrosis, liver fibrosis, pancreatic fibrosis, lung fibrosis, liver fibrosis, pulmonary fibrosis, diabetic nephropathy, metabolic syndrome, scleroderma, type 2 diabetes mellitus, acute lung injury, non-alcoholic fatty liver disease, chronic kidney disease, diabetic kidney disease, Crohn's disease, scleroderma, osteoporosis, or pulmonary hypertension; optionally for use in the treatment of Alstrom Syndrome or Idiopathic pulmonary fibrosis.

14. Use of crystalline Form ST2 of Setogepram sodium according to any one of Claims 1 to 7, in the preparation of another solid state form of Setogepram sodium, or another Setogepram salt or solid state form thereof.

15. A process for preparing a Setogepram salt or a solid state form thereof comprising preparing crystalline Form ST2 of Setogepram sodium according to any one of Claims 1 to 7, and converting it to another Setogepram salt or a solid state form thereof.

## Patentansprüche

1. Kristalline Form ST2 von Setogepram-Natrium, 2-(3-Pentylphenyl)essigsäure-Natriumsalz, **gekennzeichnet durch** Daten ausgewählt aus aus einem oder mehreren der Folgenden:
a. ein XRPD-Muster mit Peaks bei 3,6, 10,9, 19,9, 26,5 und 28,1 Grad 2-Theta ± 0,2 Grad 2-Theta oder ein XRPD-Muster mit Peaks bei 3,6, 10,9, 19,9, 26,5 und 28,1 Grad 2-Theta ± 0,2 Grad 2-Theta;
b. ein XRPD-Muster mit Peaks bei 3,6, 10,9, 19,9, 26,5 Grad 2-Theta ± 0,2 Grad 2-Theta oder ein XRPD-Muster mit Peaks bei 3,6, 10,9, 19,9, 26,5 und 28,1 Grad 2-Theta ± 0,2 Grad 2-Theta.
c. ein wie in Figur 1 dargestelltes XRPD-Muster;
d. ein Festkörper-¹³C-NMR-Spektrum mit Peaks bei 22,7, 31,3, 36,0, 125,7, 128,5 und 129,8 ppm ± 0,2 ppm;
e. ein Festkörper-¹³C-NMR-Spektrum mit den folgenden absoluten Differenzen der chemischen Verschiebung von einem Referenzpeak bei 45,4 ppm ± 2 ppm von 22,7, 14,1, 9,4, 80,3, 83,1 und 84,4 ppm ± 0,1 ppm;
f. ein Festkörper-¹³C-NMR-Spektrum, das im Wesentlichen wie in den Figuren 3a, 3b oder 3c dargestellt ist, und
g. Kombinationen aus zwei oder mehr von a, c, d, e und f; oder Kombinationen aus zwei oder mehr von b, c, d, e und f

2. Kristalline Form ST2 von Setogepram-Natrium nach Anspruch 1, **gekennzeichnet durch** ein XRPD-Muster mit Peaks bei 3,6, 10,9, 19,9, 26,5 und 28,1 Grad 2-Theta ± 0,2 Grad 2-Theta und außerdem ein, zwei, drei, vier oder fünf zusätzlichen Peaks ausgewählt aus 18,6, 21,8, 25,5, 28,1 und 29,2 Grad 2-Theta ± 0,2 Grad 2-Theta.

3. Kristalline Form ST2 von Setogepram-Natrium nach Anspruch 1, **gekennzeichnet durch** ein XRPD-Muster mit Peaks bei 3,6, 10,9, 19,9 und 26,5 Grad 2-Theta ± 0,2 Grad 2-Theta und außerdem ein, zwei, drei, vier oder fünf zusätzlichen Peaks ausgewählt aus 18,6, 21,8, 25,5, 28,1 und 29,2 Grad 2-Theta ± 0,2 Grad 2-Theta.

4. Kristalline Form ST2 von Setogepram-Natrium nach einem der Ansprüche 1, 2 oder 3, **gekennzeichnet durch** ein XRPD-Muster mit Peaks bei: 3,6, 10,9, 18,6, 19,9, 21,8, 25,5, 26,5, 28,1, 29,2 Grad 2-Theta ± 0,2 Grad 2-Theta.

5. Kristalline Form ST2 von Setogepram-Natrium nach einem der Ansprüche 1 bis 4, wobei die kristalline Form eine Hydratform ist, wobei die kristalline Form gegebenenfalls etwa 8,0 Gew.-% bis etwa 11 Gew.-% Wasser enthält oder wobei die kristalline Form ein Sesquihydrat ist.

6. Kristallines Setogepram-Natrium nach einem der Ansprüche 1 bis 5, das Folgendes enthält: Nicht mehr als etwa 20 %, nicht mehr als etwa 10 %, nicht mehr als etwa 5 %, nicht mehr als etwa 2 %, nicht mehr als etwa 1 % oder etwa 0 % aller anderen kristallinen Formen von Setogepram-Natrium.

7. Kristallines Setogepram-Natrium nach einem der Ansprüche 1 bis 6, das Folgendes enthält: Nicht mehr als etwa 20 %, nicht mehr als etwa 10 %, nicht mehr als etwa 5 %, nicht mehr als etwa 2 %, nicht mehr als etwa 1 % oder etwa 0 % amorphes Setogepram-Natrium.

8. Pharmazeutische Zusammensetzung, umfassend die kristalline Form ST2 von Setogepram-Natrium nach einem der Ansprüche 1 bis 7.

9. Verwendung der kristallinen Form ST2 von Setogepram-Natrium nach einem der Ansprüche 1 bis 7 zur Herstellung einer pharmazeutischen Zusammensetzung und/oder pharmazeutischen Formulierung, wobei die pharmazeutische Formulierung vorzugsweise eine orale Formulierung ist.

10. Pharmazeutische Formulierung, umfassend die kristalline Form ST2 von Setogepram-Natrium nach einem der Ansprüche 1 bis 7 oder eine pharmazeutische Zusammensetzung nach Anspruch 8 mit mindestens einem pharmazeutisch unbedenklichen Exzipienten.

11. Verfahren zur Herstellung einer pharmazeutischen Zusammensetzung nach Anspruch 10, bei dem man die kristalline Form ST2 von Setogepram-Natrium nach einem der Ansprüche 1 bis 7 oder eine pharmazeutische Zusammensetzung nach Anspruch 8 mit mindestens einem pharmazeutisch unbedenklichen Exzipienten kombiniert.

12. Kristalline Form ST2 von Setogepram-Natrium, nach einem der Ansprüche 1 bis 7, pharmazeutische Zusammensetzung nach Anspruch 8 oder pharmazeutische Formulierung nach Anspruch 10 zur Verwendung als Medikament.

13. Kristalline Form ST2 von Setogepram-Natrium, nach einem der Ansprüche 1 bis 7, pharmazeutische Zusammensetzung nach Anspruch 8 oder pharmazeutische Formulierung nach Anspruch 10 zur Verwendung bei der Behandlung von: Alstrom-Syndrom, idiopathischer Lungenfibrose, zystischer Fibrose, Nierenfibrose, Leberfibrose, Pankreasfibrose, Lungenfibrose, Leberfibrose, pulmonaler Fibrose, diabetischer Nephropathie, metabolischem Syndrom, Sklerodermie, Diabetes mellitus vom Typ 2, akuter Lungenschädigung, nichtalkoholischer Fettleber, chronischer Nierenerkrankung, diabetischer Nierenerkrankung, Morbus Crohn, Sklerodermie, Osteoporose oder pulmonaler Hypertonie; gegebenenfalls zur Behandlung von Alstrom-Syndrom oder idiopathischer pulmonaler Fibrose.

14. Verwendung der kristallinen Form ST2 von Setogepram-Natrium nach einem der Ansprüche 1 bis 7 zur Herstellung einer anderen festen Form von Setogepram-Natrium oder eines anderen Setogepram-Salzes oder einer festen Form davon.

15. Verfahren zur Herstellung eines Setogepram-Salzes oder einer festen Form davon, umfassend die Herstellung der kristallinen Form ST2 von Setogepram-Natrium nach einem der Ansprüche 1 bis 7 und deren Umwandlung in ein anderes Setogepram-Salz oder eine feste Form davon.

## Revendications

1. Forme cristalline ST2 du Sétogépram sodique, sel de sodium de l'acide 2-(3-pentylphényl)acétique, qui est **caractérisée par** des données choisies parmi un ou plusieurs des éléments suivants :
a. un diagramme de XRPD ayant des pics à 3,6, 10,9, 19,9, 26,5 et 28,1 degrés 2-thêta ± 0,2 degré 2-thêta ou un diagramme de XRPD ayant des pics à 3,6, 10,9, 19,9, 26,5 et 28,1 degrés 2-thêta ± 0,2 degré 2-thêta ;
b. un diagramme de XRPD ayant des pics à 3,6, 10,9, 19,9, 26,5 degrés 2-thêta ± 0,2 degré 2-thêta ou un diagramme de XRPD ayant des pics à 3,6, 10,9, 19,9, 26,5 et 28,1 degrés 2-thêta ± 0,2 degré 2-thêta ;
c. un diagramme de XRPD tel que représenté sur la Figure 1 ;
d. un spectre de RMN ¹³C à l'état solide ayant des pics à 22,7, 31,3, 36,0, 125,7, 128,5 et 129,8 ppm ± 0,2 ppm ;
e. un spectre de RMN ¹³C à l'état solide présentant les différences absolues de déplacements chimiques suivantes à partir d'un pic de référence à 45,4 ppm ± 2 ppm de 22,7, 14,1, 9,4, 80,3, 83,1 et 84,4 ppm ± 0,1 ppm ;
f. un spectre de RMN ¹³C à l'état solide sensiblement tel que représenté sur les Figures 3a, 3b ou 3c ; et
g. des combinaisons de deux éléments suivants ou plus : a, c, d, e et f ; ou des combinaisons de deux éléments suivants ou plus : b, c, d, e et f.

2. Forme cristalline ST2 du Sétogépram sodique selon la revendication 1, qui est **caractérisée par** un diagramme de XRPD ayant des pics à 3,6, 10,9, 19,9, 26,5 et 28,1 degrés 2-thêta ± 0,2 degré 2-thêta, et ayant également un, deux, trois, quatre ou cinq pics supplémentaires sélectionnés parmi 18,6, 21,8, 25,5, 28,1 et 29,2 degrés 2-thêta ± 0,2 degré 2-thêta.

3. Forme cristalline ST2 du Sétogépram sodique selon la revendication 1, qui est **caractérisée par** un diagramme de XRPD ayant des pics à 3,6, 10,9, 19,9 et 26,5 degrés 2-thêta ± 0,2 degré 2-thêta, et ayant également un, deux, trois, quatre ou cinq pics supplémentaires sélectionnés parmi 18,6, 21,8, 25,5, 28,1 et 29,2 degrés 2-thêta ± 0,2 degré 2-thêta.

4. Forme cristalline ST2 du Sétogépram sodique selon l'une quelconque des revendications 1, 2 ou 3, qui est **caractérisée par** un diagramme de XRPD ayant des pics à : 3,6, 10,9, 18,6, 19,9, 21,8, 25,5, 26,5, 28,1, 29,2 degrés 2-thêta ± 0,2 degré 2-thêta.

5. Forme cristalline ST2 du Sétogépram sodique selon l'une quelconque des revendications 1 à 4, dans laquelle ladite forme cristalline est une forme d'hydrate, éventuellement dans laquelle la forme cristalline contient environ 8,0 % à environ 11 % (p/p) d'eau, ou dans laquelle la forme cristalline est un sesquihydrate.

6. Forme cristalline du Sétogépram sodique selon l'une quelconque des revendications 1 à 5, qui ne contient : pas plus d'environ 20 %, pas plus d'environ 10 %, pas plus d'environ 5 %, pas plus d'environ 2 %, pas plus d'environ 1 % ou qui contient environ 0 % de quelconques autres formes cristallines du Sétogépram sodique.

7. Forme cristalline du Sétogépram sodique selon l'une quelconque des revendications 1 à 6, qui ne contient : pas plus d'environ 20 %, pas plus d'environ 10 %, pas plus d'environ 5 %, pas plus d'environ 2 %, pas plus d'environ 1 % ou qui contient environ 0 % de Sétogépram sodique amorphe.

8. Composition pharmaceutique comprenant une forme cristalline ST2 du Sétogépram sodique selon l'une quelconque des revendications 1 à 7.

9. Utilisation d'une forme cristalline ST2 du Sétogépram sodique selon l'une quelconque des revendications 1 à 7 pour la préparation d'une composition pharmaceutique et/ou d'une formulation pharmaceutique, de préférence dans laquelle la formulation pharmaceutique est une formulation orale.

10. Formulation pharmaceutique comprenant une forme cristalline ST2 du Sétogépram sodique selon l'une quelconque des revendications 1 à 7, ou une composition pharmaceutique selon la revendication 8, avec au moins un excipient pharmaceutiquement acceptable.

11. Procédé de préparation d'une formulation pharmaceutique selon la revendication 10, comprenant la combinaison d'une forme cristalline ST2 du Sétogépram sodique selon l'une quelconque des revendications 1 à 7 ou d'une composition pharmaceutique selon la revendication 8, avec au moins un excipient pharmaceutiquement acceptable.

12. Forme cristalline ST2 du Sétogépram sodique selon l'une quelconque des revendications 1 à 7, composition pharmaceutique selon la revendication 8 ou formulation pharmaceutique selon la revendication 10, pour une utilisation en tant que médicament.

13. Forme cristalline ST2 du Sétogépram sodique, selon l'une quelconque des revendications 1 à 7, composition pharmaceutique selon la revendication 8, ou formulation pharmaceutique selon la revendication 10, pour une utilisation dans le traitement de : syndrome d'Alström, fibrose pulmonaire idiopathique, fibrose kystique, fibrose rénale, fibrose hépatique, fibrose pancréatique, fibrose pulmonaire, fibrose hépatique, fibrose pulmonaire, néphropathie diabétique, syndrome métabolique, sclérodermie, diabète sucré de type 2, lésion pulmonaire aiguë, stéatose hépatique non alcoolique, maladie rénale chronique, maladie rénale diabétique, maladie de Crohn, sclérodermie, ostéoporose ou hypertension pulmonaire ; éventuellement pour une utilisation dans le traitement du syndrome d'Alström ou de la fibrose pulmonaire idiopathique.

14. Utilisation d'une forme cristalline ST2 du Sétogépram sodique selon l'une quelconque des revendications 1 à 7, dans la préparation d'une autre forme du Sétogépram sodique à l'état solide, ou d'un autre sel de Sétogépram ou d'une forme de celui-ci à l'état solide.

15. Procédé de préparation d'un sel de Sétogépram ou d'une forme de celui-ci à l'état solide, comprenant la préparation de la forme cristalline ST2 du Sétogépram sodique selon l'une quelconque des revendications 1 à 7, et sa conversion en un autre sel de Sétogépram ou une forme de celui-ci à l'état solide.
